# EUROPEAN PATENT APPLICATION

(11) **EP 4 736 992 A1**
(43) Date of publication of application: **06.05.2026**
(21) Application number: 25212169.4
(22) Date of filing: 29.10.2025
(51) Int. Cl.: B01D 53/34, B01D 53/52, B01D 53/82, B01D 53/96, C10L 3/10, C12M 1/00

(54) **SYSTEM AND METHOD FOR PERFORMING A REGENERATIVE DESULFURIZATION OF RAW BIOGAS**

(30) Priority: 01.11.2024 US 202463714889 P; 16.01.2025 US 202563745850 P
(71) Applicant: Obeo Biogas, Montreal, QC H3A 3G3 (CA)
(72) Inventor: LAVOIE, Renaud, Laval, Québec H7Y 2A7 (CA); DOAN, Duy Tuan, Trois-Rivières, Québec G8Z 1X5 (CA); VILLEMURE, Tristan, Beauharnois, Québec J6N 0L8 (CA)
(74) Representative: Riechelmann & Carlsohn Patentanwälte PartG mbB

(57) **Abstract**

System and method for performing a regenerative desulfurization of raw biogas. Raw biogas comprising hydrogen sulfide is injected in a container containing a material adapted for capturing and converting into solid sulfur the hydrogen sulfide. Biogas without hydrogen sulfide is extracted from the container, the hydrogen sulfide being captured and converted into the solid sulfur by the material. The injection of the raw biogas comprising the hydrogen sulfide in the container is then stopped. A regeneration gas heated at a predetermined temperature is injected in the container, the temperature allowing extraction of the sulfur from the material in the form of sulfur compound(s). The regeneration gas and the sulfur compound(s) are extracted from the container. The injection of the regeneration gas in the container is then stopped. A control device controls the execution of the regenerative desulfurization, using collected data representative of operating conditions of the system.

## Description

### TECHNICAL FIELD

The present disclosure relates to the field of biogas-based energy generation. More specifically, the present disclosure relates to a system and method for performing a regenerative desulfurization of raw biogas.

### BACKGROUND

Raw biogas can be produced by the decomposition of various organic matters, one of them being cattle manure (e.g. cow manure). Equipment adapted for raw biogas production (usually an anaerobic digester) can be deployed at a farm where organic matter suitable for raw biogas production is a by-product of the farm activities. The digester is used to process the organic matter (e.g. cattle manure) to obtain the raw biogas.

The quality of raw biogas is not comparable to natural gas. The raw biogas (also referred to as digester gas) contains methane, but also carbon dioxide gas, hydrogen sulfide, ammoniac, hydrogen, oxygen, water vapor, etc. In particular, the concentration of methane in raw biogas is lower than in natural gas. Furthermore, different types of impurities are present in raw biogas, which are not present in natural gas (or at least at lower levels). Consequently, an upgrading process is generally implemented to upgrade raw biogas into biomethane having a quality similar to (or at least approaching) pipeline quality renewable natural gas (RNG). Upgrading equipment can also be deployed directly at the farm to perform the upgrading process.

The present disclosure focuses on the capture and conversion of hydrogen sulfide (H₂S) present in raw biogas into a sulfur element. Several processes for performing this operation are already known in the art. For example, iron(III) chloride (also referred to as ferric chloride) in liquid form is added directly to the digester. In another example, raw biogas exiting the digester is treated with activated carbon for the capture and conversion of hydrogen sulfide. The adsorption capacity is approximately 25%, meaning that one kilogram of activated carbon is capable of adsorbing 250 grams of sulfur under a solid form. In still another example, raw biogas exiting the digester is treated with iron(III) oxide-hydroxide FeO(OH) (also referred to as ferric oxyhydroxide) for the capture of the hydrogen sulfide. The adsorption capacity is approximately 50% to 60%, meaning that one kilogram of ferric oxyhydroxide is capable of capturing 500 to 600 grams of of hydrogen sulfide and converting it into a sulfur element under a solid form, which can be disposed of in an environmentally friendly way.

However, the aforementioned processes have several drawbacks, including the need to dispose of the exhausted material used for capturing hydrogen sulfide from the raw biogas, once the material becomes saturated with sulfur.

There is therefore a need for a new system and method for performing a regenerative desulfurization of raw biogas.

### SUMMARY

According to a first aspect, the present disclosure relates to a system adapted for performing a regenerative desulfurization of raw biogas. The system comprises a container containing a material adapted for capturing and converting into solid sulfur hydrogen sulfide. The system comprises means for injecting the raw biogas comprising the hydrogen sulfide in the container. The system comprises means for extracting biogas without hydrogen sulfide from the container, the hydrogen sulfide being captured and converted into the solid sulfur by the material. The system comprises means for injecting a regeneration gas heated at a predetermined temperature in the container, the predetermined temperature allowing extraction of the sulfur from the material in the form of one or more sulfur compounds. The system comprises means for extracting the regeneration gas and the one or more sulfur compounds from the container.

In a particular aspect, the system further comprises a control device adapted for collecting data representative of operating conditions of the system, executing a control algorithm using the collected data for determining at least one operating parameter of a component of the system, and applying the at least one operating parameter to the component.

In a particular embodiment, the operating parameter comprises at least one of the following: initiation or interruption of the injection of the raw biogas with hydrogen sulfide in the container, flow rate of the raw biogas with hydrogen sulfide injected in the container, initiation or interruption of the extraction of biogas without hydrogen sulfide from the container, flow rate of the biogas without hydrogen sulfide extracted from the container, initiation or interruption of the injection of heated regeneration gas in the container, flow rate of the heated regeneration gas injected in the container, initiating or stopping the extraction of the regeneration gas and the one or more sulfur compounds from the container.

In another particular embodiment, the data representative of operating conditions comprise at least one of the following: flow rate of raw biogas with hydrogen sulfide injected in the container, concentration in at least one of hydrogen sulfide, oxygen, ammonia, methane, carbon dioxide and humidity of the raw biogas injected in the container, flow rate of biogas extracted from the container, concentration in hydrogen sulfide of the biogas extracted from the container, estimation of a concentration of sulfur in the material, flow rate of regeneration gas injected in the container, concentration in nitrogen of the regeneration gas injected in the container, concentration in oxygen of the regeneration gas injected in the container, temperature of the regeneration gas injected in the container, flow rate of the one or more sulfur compound extracted from the container, concentration of the one or more sulfur compound extracted from the container, quantity of solid sulfur extracted from the container, concentration in oxygen of the regeneration gas extracted from the container, concentration in other exhaust gas extracted from the container, temperature inside the container, pressure inside the container, humidity inside the container, and weather conditions.

In another particular aspect, the system comprises a first container consisting of the aforementioned container. The system further comprises a second container containing the material adapted for capturing and converting into solid sulfur the hydrogen sulfide. The system further comprises means for injecting the raw biogas comprising the hydrogen sulfide in the second container. The system further comprises means for extracting biogas without hydrogen sulfide from the second container, the hydrogen sulfide being captured and converted into the solid sulfur by the material in the second container. The system further comprises means for injecting the regeneration gas heated at the predetermined temperature in the second container, the predetermined temperature allowing extraction of the sulfur from the material in the second container in the form of one or more sulfur compounds. The system further comprises means for extracting the regeneration gas and the one or more sulfur compounds from the second container. The raw biogas with hydrogen sulfide is injected in the container while the regeneration gas heated at the predetermined temperature is injected in the second container, and the raw biogas with hydrogen sulfide is injected in the second container while the regeneration gas heated at the predetermined temperature is injected in the first container.

In still another particular aspect, the material is a porous carbonaceous solid material impregnated with metallic nanoparticles.

In yet another particular aspect, the regeneration gas comprises nitrogen mixed with at least one of oxygen and carbon dioxide.

In another particular aspect, the one or more sulfur compounds comprise at least one of the following: solid sulfur, sulfur dioxide, sulfurous acid, sulfur trioxide, hydrogen sulfide, and sulfuric acid.

According to a second aspect, the present disclosure relates to a method for performing a regenerative desulfurization procedure of raw biogas. The method comprises (i) injecting the raw biogas comprising hydrogen sulfide in a container, the container containing a material adapted for capturing and converting into solid sulfur the hydrogen sulfide. The method comprises (ii) extracting biogas without hydrogen sulfide from the container, the hydrogen sulfide being captured and converted into the solid sulfur by the material. The method comprises (iii) stopping the injection of the raw biogas comprising the hydrogen sulfide in the container. The method comprises (iv) injecting a regeneration gas heated at a predetermined temperature in the container, the predetermined temperature allowing extraction of the sulfur from the material in the form of one or more sulfur compounds. The method comprises (v) extracting the regeneration gas and the one or more sulfur compounds from the container. The method comprises (vi) stopping the injection of the regeneration gas in the container.

In a particular aspect, steps (i) to (vi) are repeated several times.

In another particular aspect, the method of further comprises collecting by a control device data representative of operating conditions of the regenerative desulfurization procedure, executing by the control device a control algorithm using the collected data for determining at least one operating parameter of the regenerative desulfurization procedure, and applying the at least one operating parameter to a component implementing the regenerative desulfurization procedure.

In a particular embodiment, the operating parameter comprises at least one of the following: initiation or interruption of the injection of the raw biogas with hydrogen sulfide in the container, flow rate of the raw biogas with hydrogen sulfide injected in the container, initiation or interruption of the extraction of biogas without hydrogen sulfide from the container, flow rate of the biogas without hydrogen sulfide extracted from the container, initiation or interruption of the injection of heated regeneration gas in the container, flow rate of the heated regeneration gas injected in the container, initiating or stopping the extraction of the regeneration gas and the one or more sulfur compounds from the container.

In another particular embodiment, the data representative of operating conditions comprise at least one of the following: flow rate of raw biogas with hydrogen sulfide injected in the container, concentration in at least one of hydrogen sulfide, oxygen, ammonia, methane, carbon dioxide and humidity of the raw biogas injected in the container, flow rate of biogas extracted from the container, concentration in hydrogen sulfide of the biogas extracted from the container, estimation of a concentration of sulfur in the material, flow rate of regeneration gas injected in the container, concentration in nitrogen of the regeneration gas injected in the container, concentration in oxygen of the regeneration gas injected in the container, temperature of the regeneration gas injected in the container, flow rate of the one or more sulfur compound extracted from the container, concentration of the one or more sulfur compound extracted from the container, quantity of solid sulfur extracted from the container, concentration in oxygen of the regeneration gas extracted from the container, concentration in other exhaust gas extracted from the container, temperature inside the container, pressure inside the container, humidity inside the container, and weather conditions.

In still another particular aspect, the method of further comprises collecting by a control device data representative of operating conditions of the regenerative desulfurization procedure, determining by the control device during the execution of steps (i) and (ii) based on the collected data that the material is saturated with sulfur, and enforcing by the control device the interruption of steps (i) and (ii) and the initiation of steps (iii), (iv) and (v).

In yet another particular aspect, the method further comprises collecting by a control device data representative of operating conditions of the regenerative desulfurization procedure, determining by the control device during the execution of steps (iv) and (v) based on the collected data that regeneration of the material is completed, and enforcing by the control device the interruption of steps (iv) and (v) and the initiation of steps (vi), (i) and (ii).

In another particular aspect, the method further comprises collecting by a control device data representative of operating conditions of the regenerative desulfurization procedure, and determining by the control device based on the collected data that material needs to be added to the container to replace material consumed by the regenerative desulfurization procedure.

In still another particular aspect, the method further comprises, simultaneously to steps (i) and (ii): injecting the regeneration gas heated at the predetermined temperature in a second container, the second container containing the material with deposited sulfur; and extracting from the second container the regeneration gas and one or more sulfur compounds generated by the extraction of the sulfur from the material of the second container under the action of the heated regeneration gas.

In yet another particular aspect, the method further comprises, simultaneously to steps (iv) and (v): injecting the raw biogas comprising the hydrogen sulfide in a second container, the second container containing the material adapted for capturing and converting into solid sulfur the hydrogen sulfide; and extracting biogas without hydrogen sulfide from the second container, the hydrogen sulfide being captured and converted into the solid sulfur by the material of the second container.

In another particular aspect, the material is a porous carbonaceous solid material impregnated with metallic nanoparticles.

In still another particular aspect, the regeneration gas comprises nitrogen mixed with at least one of oxygen and carbon dioxide.

In yet another particular aspect, the one or more sulfur compounds comprise at least one of the following: solid sulfur, sulfur dioxide, sulfurous acid, sulfur trioxide, hydrogen sulfide, and sulfuric acid.

### BRIEF DESCRIPTION OF THE DRAWINGS

Embodiments of the disclosure will be described by way of example only with reference to the accompanying drawings, in which:
Figure 1 illustrates a biogas-based energy generation system;
Figures 2, 3 and 4 illustrate a regenerative hydrogen sulfide capture system;
Figures 5, 6, 7 and 8 illustrate another implementation of a regenerative hydrogen sulfide capture system;
Figure 9 illustrates still another implementation of a regenerative hydrogen sulfide capture system;
Figure 10 illustrates the fuel cell of Figure 9 being adapted to also operate in electrolysis mode;
Figure 11 illustrates a method for performing a regenerative desulfurization of raw biogas;
Figure 12 illustrates a control device controlling operations of the aforementioned regenerative hydrogen sulfide capture systems;
Figure 13 is a schematic representation of components of the control device illustrated in Figure 12;
Figure 14 illustrates a method for controlling a regenerative capture of hydrogen sulfide present in raw biogas implemented by the control device of Figures 12 and 13; and
Figure 15 illustrates another method for performing a regenerative desulfurization of raw biogas.

### DETAILED DESCRIPTION

The foregoing and other features will become more apparent upon reading of the following non-restrictive description of illustrative embodiments thereof, given by way of example only with reference to the accompanying drawings. Like numerals represent like features on the various drawings.

Various aspects of the present disclosure generally address the implementation of a system and procedure for performing a regenerative desulfurization of raw biogas. More specifically, several implementations of a regenerative hydrogen sulfide capture system are provided. The system relies on a material adapted for capturing and converting into solid sulfur hydrogen sulfide present in raw biogas. Furthermore, the system is adapted for operating in two complementary modes. A desulfurization mode, where the hydrogen sulfide is captured and converted into solid sulfur by the material. And a regeneration mode, where the sulfur is extracted from the material by injection of a heated regeneration gas. Additionally, one or more control algorithms are used for controlling operations of the system based on various operating conditions of the system (collected for instance via sensors).

The following terminology is used throughout the present disclosure:
Sulfur compound: any compound in solid, liquid or gaseous form comprising sulfur. The compound may comprise only sulfur in solid form.
Raw biogas and biogas: raw biogas needs to go through an upgrading process (e.g. filtering, etc.) before being used for producing energy. The terminology biogas is used for the result of the upgrading process of the raw biogas. The biogas is (for example) used for producing energy.

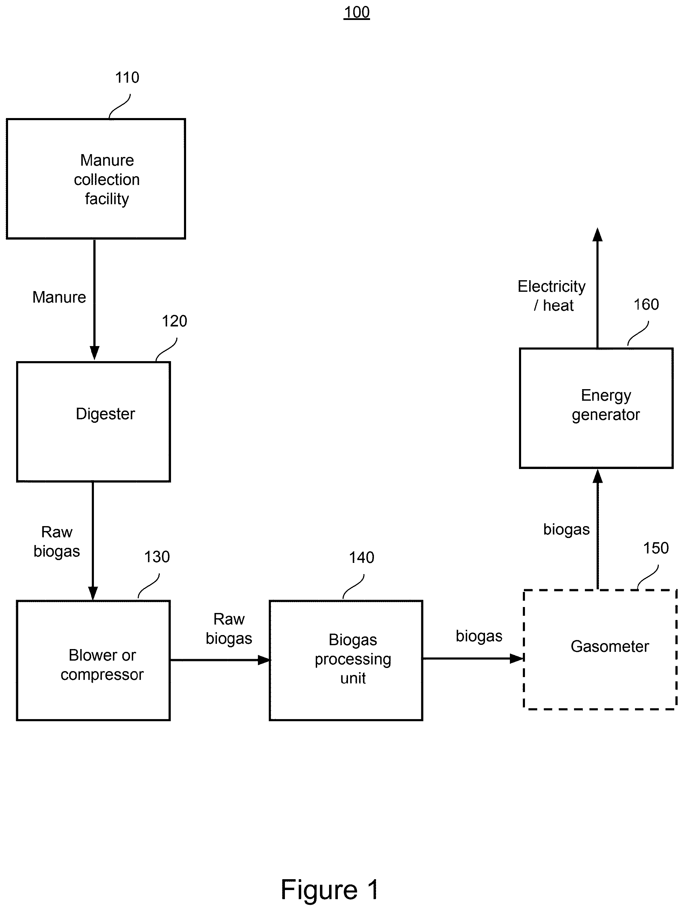

Referring now to **Figure 1****,** a biogas-based energy generation system 100 is represented. The system 100 comprises several equipment involved in a biogas-based energy generation process. Each equipment implements one or more steps of this process. The system 100 represented in Figure 1 is for illustration purposes only. Other implementations of the system 100 are also within the scope of the present disclosure.

The system 100 comprises a manure collection facility 110. Manure produced by cattle (e.g. cow, pig, sheep, etc.) living on the farm is collected at the manure collection facility 110. The collected manure is transferred to a digester 120. Optionally, the manure collection facility 110 comprises a storage unit (e.g. tanks), where collected manure is temporarily stored before being transferred to the digester 120. The manure transferred from the manure collection facility 110 to the digester 120 is usually also referred to as feedstock.

The system 100 comprises the digester 120. A digester is an equipment well known in the art. The digester 120 processes the manure received from the manure collection facility 110 to produce raw biogas. The digester 120 operates at a specific temperature or range of temperatures (e.g. 30 to 40 degrees Celsius for a mesophilic digestion and 50 to 60 degrees Celsius for a thermophilic digestion). Therefore, heating means (not represented in Figure 1) are used for maintaining the digester 120 at the proper operating temperature or range of temperatures.

Optionally, the system 100 comprises a blower or compressor 130 operating at low or high pressure. The blower or compressor 130 is responsible for providing a constant pressure to the raw biogas entering the biogas processing unit 140

The system 100 comprises a biogas processing unit 140, to upgrade the raw biogas into biogas (also referred to as biomethane) via one or more sub-processes, each sub-process being implemented by one or more dedicated components (not represented in Figure 1 for simplification purposes).

More specifically, the biogas processing unit 140 implements at least one of the following sub-processes: undesired gas removal (e.g. hydrogen sulfide (H₂S) removal, ammoniac removal, etc.), water (H₂O) removal, gas drying, gas polishing, etc. The present disclosure focuses on the sub-process consisting of performing hydrogen sulfide (H₂S) removal.

Optionally, the system 100 comprises a gasometer 150 for accumulating the biogas before it is used for producing energy. A gasometer is also well known in the art. The biogas stored in the gasometer 150 is compressed, in order to increase the quantity of biogas that can be stored. The gasometer 150 can be dimensioned to store multiple hours of biogas production (by the digester 120).

The system 100 comprises an energy generator 160 for generating energy using the biogas stored in the gasometer 150 (or the biogas directly received from the biogas processing unit 140). Examples of energy generators include a combined heat and power (CHP) generator that burns the biogas to generate electricity and produces heat as a by-product, the combination of a reformer (which converts the biogas into syngas (also referred to as synthesis gas), the syngas comprising a mixture of hydrogen and carbon monoxide) and a fuel cell that also generates electricity (using the syngas) and produces heat as a by-product, etc. Other types of equipment may be used in place of (or in combination with) the energy generator 160, such as: an upgrading device adapted for converting the biogas into renewable natural gas, a standalone reformer (without the fuel cell) for converting the biogas into syngas, etc. Consequently, the present disclosure applies to any type of equipment (e.g. energy generator 160) adapted to use the biogas for directly generating bioenergy (e.g. electricity, heat, etc.) or adapted to convert the biogas into another gaseous compound (e.g. syngas, hydrogen, renewable natural gas, etc.) which is used later to generate bioenergy.

If electrical power is produced by the energy generator 160, it can be injected in the electrical grid of an operator and / or used locally (e.g. to provide electrical power to equipment operated at the farm). Optionally, heat produced by the energy generator 160 can be used for heating other equipment (e.g. the digester 120, etc.).

In the case where instead of the energy generator 160, an equipment uses the biogas to generate a gaseous compound (e.g. natural gas, syngas or hydrogen) that is not directly used locally, the generated gaseous compound (e.g. natural gas, syngas or hydrogen) can be transported and used by any facility (e.g. a factory) or device capable of using the gaseous compound (e.g. natural gas, syngas or hydrogen) as a source of energy. In this configuration, heat produced in the process of generating the gaseous compound is also optionally used for heating other equipment (e.g. the digester 120, etc.).

The system 100 has been described as having the capability to process manure in the digester 120 to produce raw biogas. However, the system 100 is not limited to the processing of manure; but can be generalized to the processing by the digester 120 of any organic matter which can be turned into raw biogas.

Furthermore, the present disclosure is not limited to the digester 120 used to process organic matter (e.g. cattle manure) to obtain raw biogas. Other sources of raw biogas can also be processed by the biogas processing unit 140 (e.g. raw biogas generated at a landfill, raw biogas generated by the decomposition of organic waste (e.g. food waste), raw biogas generated by water treatment, etc.). These other sources are not represented in Figure 1 for simplification purposes.

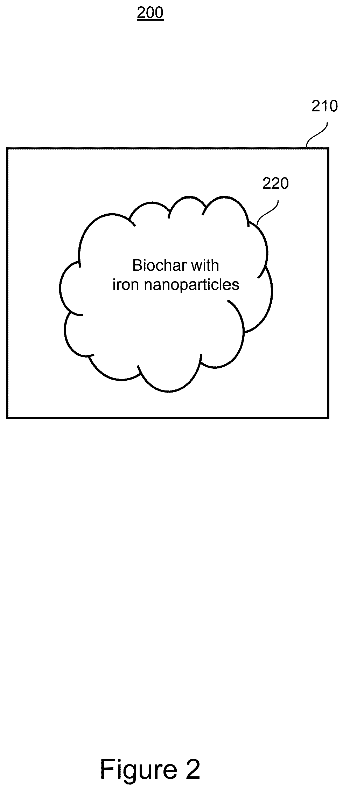

Referring now concurrently to **Figures 1****,** **2****,** **3** **and** **4****,** a regenerative hydrogen sulfide capture sub-system 200 for removing hydrogen sulfide contained in raw biogas is represented in Figures 2-4. The sub-system 200 comprises component(s) of the biogas processing unit 140 illustrated in Figure 1, which are dedicated to the removal of hydrogen sulfide. For simplification purposes, the sub-system 200 will be simply referred to as the system 200 in the following.

Referring more specifically to **Figure 2****,** the system 200 comprises a container 210 containing a material 220 adapted to capture and convert into solid sulfur the hydrogen sulfide (generally referred to as desulfurization agent). Examples of containers include, without limitations: a tank, etc.

The material 220 is made of biochar with nanoparticles of iron. Biochar is a porous carbonaceous solid material well known in the art, obtained by the thermal degradation of biomass in oxygen starved conditions. The material 220 can be prepared by impregnating the biochar with the nanoparticles of iron (e.g. in a basin, via deposition, etc.). The material 220 is not limited to biochar, but includes any porous carbonaceous solid material adapted to be impregnated with nanoparticles of iron. The material 220 will be referred to as pure material when it is loaded in the container 210, before any chemical reaction occurs with the material 220.

When the material 220 is put in presence of hydrogen sulfide, a chemical reaction occurs between the nanoparticles of iron and the hydrogen sulfide (a deposition of the sulfur contained in the hydrogen sulfide), resulting in the decomposition of the hydrogen sulfide and the capture in the material 220 of sulfur in the (solid) form of iron(III) sulfide (Fe₂S₃). Water (H₂O) is also created in the process.

For example, the following chemical reaction (A) occurs: Fe₂O₃ + 3 H₂S -> Fe₂S₃ + 3 H₂O
The adsorption capacity is 40 to 60 %, meaning that one kilogram of material 220 is capable of capturing 400 to 600 grams of hydrogen sulfide and converting it to iron (III) sulfide through chemical reaction (A).

Instead of iron, the material 220 (e.g. biochar) may be impregnated with other types of metallic nanoparticles (e.g. Ni, Co, Cu, Ru, Rh, Pt, Pd) capable of inducing a chemical reaction of deposition of the hydrogen sulfide.

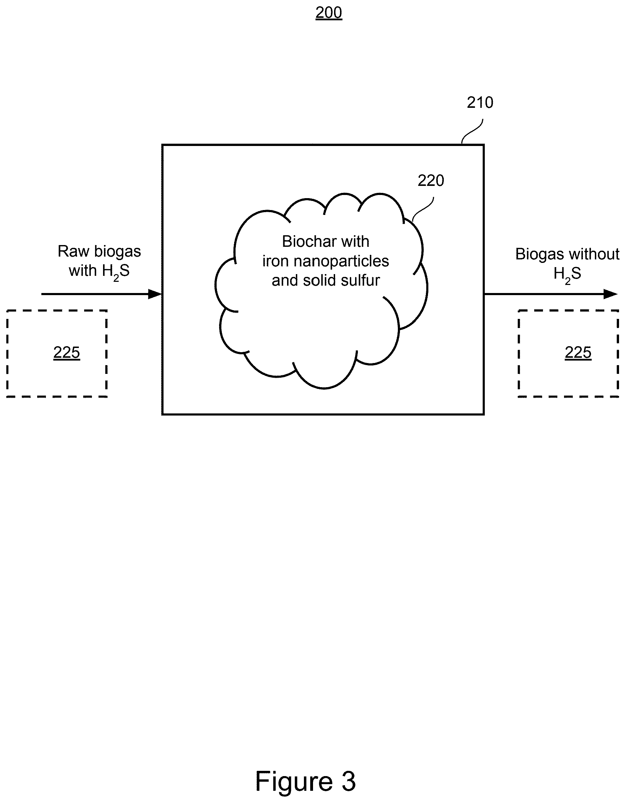

Referring more specifically to **Figure 3****,** a first phase of the hydrogen sulfide removal process is illustrated. Raw biogas with hydrogen sulfide is injected in the container 210 and chemical reaction (A) occurs, resulting in the capture of sulfur in solid form (Fe₂S₃) by material 220. Biogas without hydrogen sulfide is extracted from the container 210.

The extracted biogas may still contain traces of hydrogen sulfide, but at a concentration that is considered acceptable for further usage of the biogas. The water (H₂O) produced by chemical reaction (A) is adsorbed on the material 220.

The extracted biogas is directly used for generating energy (e.g. stored in the gasometer 150 before being used by the energy generator 160, as illustrated in Figure 1). Alternatively, the extracted biogas needs to go through one or more additional upgrading processes before being used for generating energy.

Once the material 220 becomes saturated with sulfur (when the previously mentioned adsorption capacity of 40 to 60% is reached), hydrogen sulfide can no longer be removed from the raw biogas entering the container 210. At this point, the injection of raw biogas in the container 210 is stopped.

With existing solutions implementing the capture of hydrogen sulfide, the material 220 saturated with sulfur is taken out of the container 210 and disposed of. A new batch of pure material 220 is loaded in the container 210 (as illustrated in Figure 2) and the process of capturing hydrogen sulfide is restarted (as illustrated in Figure 3). The present disclosure introduces a new mechanism allowing regenerative hydrogen sulfide capture, as described in the following.

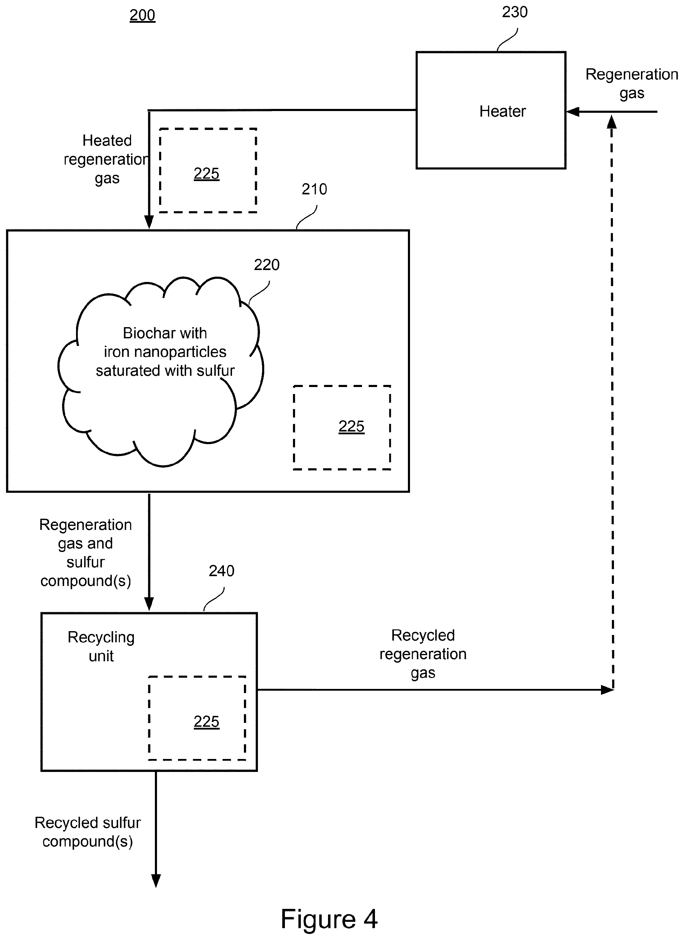

Referring more specifically to **Figure 4****,** a second phase of the hydrogen sulfide removal process is illustrated, when the material 220 is saturated with sulfur.

A regeneration gas is injected in the container 210 (the raw biogas is not injected in the container 210 during this second phase). The source of regeneration gas (e.g. a container) is not represented in Figure 4 for simplification purposes. For example, the regeneration gas consists of nitrogen (N₂) mixed with 2% (this percentage may vary) in volume of oxygen (O₂). However, other types of regeneration gas may be used in place of nitrogen. For instance, carbon dioxide (CO₂) can also be used as the regeneration gas. Usually, the biogas processing unit 140 of Figure 1 implements a procedure for extracting the carbon dioxide present in the biogas. This procedure may be implemented in different ways well known in the art. The carbon dioxide extracted from the biogas can be used as the regeneration gas.

The regeneration gas (e.g. the mixture of nitrogen and oxygen) is heated by a heater 230 to a predetermined temperature, before being injected in the container 210. The predetermined temperature is selected to trigger a chemical reaction that extracts the sulfur from the material 220 in the form of one or more sulfur compounds. The one or more sulfur compounds include at least one of the following: solid sulfur, sulfur dioxide, sulfurous acid, sulfur trioxide, sulfuric acid, hydrogen sulfide, etc.

The predetermined temperature is for example substantially 600 degrees Celsius, which allows the sulfur extraction to occur, while preserving the structure of the porous carbonaceous solid material (e.g. biochar) of the material 220. However, the value of the predetermined temperature may vary, based on the type of regeneration gas being used, the type of sulfur compound(s) being extracted from the material 220, etc.

The sulfur compound(s) and the regeneration gas are extracted from the container 210, and further injected in a recycling unit 240. The recycling unit 240 comprises one or more components (e.g. scrubber, decanter, etc.) adapted for performing various types of recycling operations. The recycling operations implemented by the recycling unit 240 are well known in the art and out of the scope of the present disclosure.

The one or more sulfur compound(s) injected in the recycling unit 240 are recycled according to a recycling process adapted to each type of sulfur compound. The recycled sulfur compound(s) are in a form that can be safely disposed of.

The regeneration gas injected in the recycling unit 240 is recycled (e.g. purified from other gasses). Optionally, the recycled regeneration gas is sent to the heater 230, to be reheated at the predetermined temperature and reinjected in the container 210. Alternatively, the recycled regeneration gas is simply disposed of in a manner which is out of the scope of the present disclosure.

At the end of this second phase, the material 220 only contains a residual quantity of solid sulfur and is substantially in the state illustrated in Figure 2. The second phase is stopped and the first phase illustrated in Figure 3 is resumed. Optionally, a cooling gas is injected in the container 210 at the end of the second phase, to cool the heated material 220. The cooling gas is the regeneration gas injected at a lower temperature or another gas.

The source of regeneration gas can be the air from the surrounding environment. For example, a nitrogen generator is used to produce a nitrogen gas flow at a purity higher than 98.5%. The nitrogen gas flow is mixed with filtered air (e.g. at a ratio of 9 to 1 for nitrogen to air) to obtain a regeneration gas with a concentration of 2 % in oxygen (of the total volume of the regeneration gas). The concentration of oxygen can be varied from 2 to 5 %. Elemental sulfur is generated in vapor phase in the container 210, extracted from the container 210, and further condensed by the recycling unit 240 (e.g. by using a heat exchanger with cool to cold air from the surrounding).

After each sequence of phase 1 followed by phase 2, the material loses around 3 to 5% of its mass of porous carbonaceous solid material (e.g. biochar). At some point (e.g. after 3 or 4 cycles), the entire process is interrupted and a quantity of pure material 220 is added to the container 210, to compensate for the loss of material 220. In an exemplary implementation, the number of cycles before adding pure material 220 is calculated based on a predetermined estimation of the mass of material lost (e.g. 3 to 5%) at each cycle and a maximum threshold for the loss of mass (e.g. 20%)). Alternatively, pure material is simply added after each cycle.

The design of a container 210 adapted for injecting gas therein and extracting gas therefrom is well known in the art and out of the scope of the present disclosure. An exemplary implementation relies on inlets for gas injection and outlets for gas extraction. Furthermore, any type of adapted opening may be used for loading the material 220 inside the container 210.

The system 200 further comprises a control device (not represented in Figures 2-4 for simplification purposes) adapted for controlling operations of the container 210. The control device is integrated to the container 210 or is a standalone control device adapted for remotely controlling operations of the container 210.

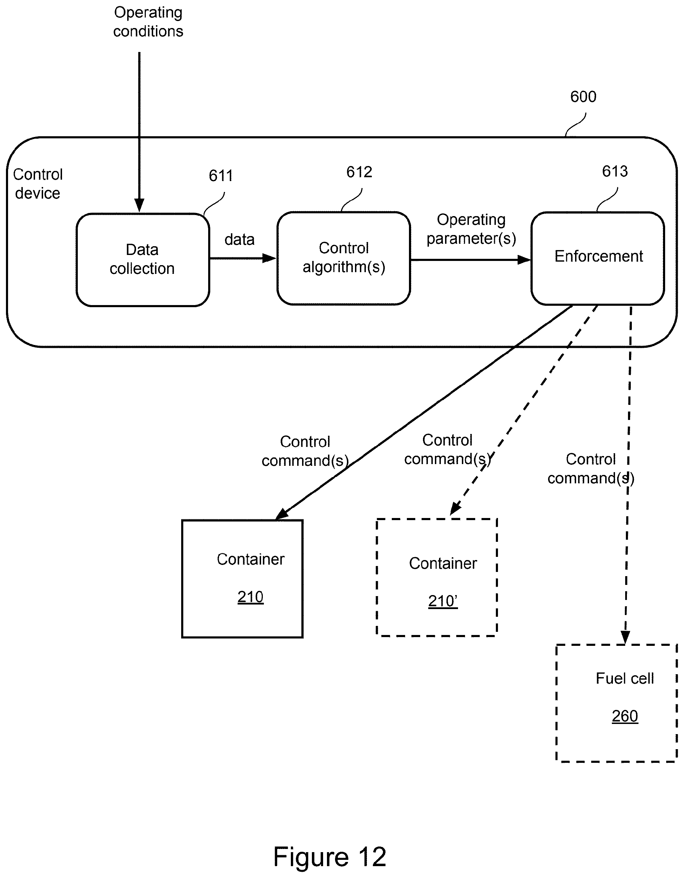

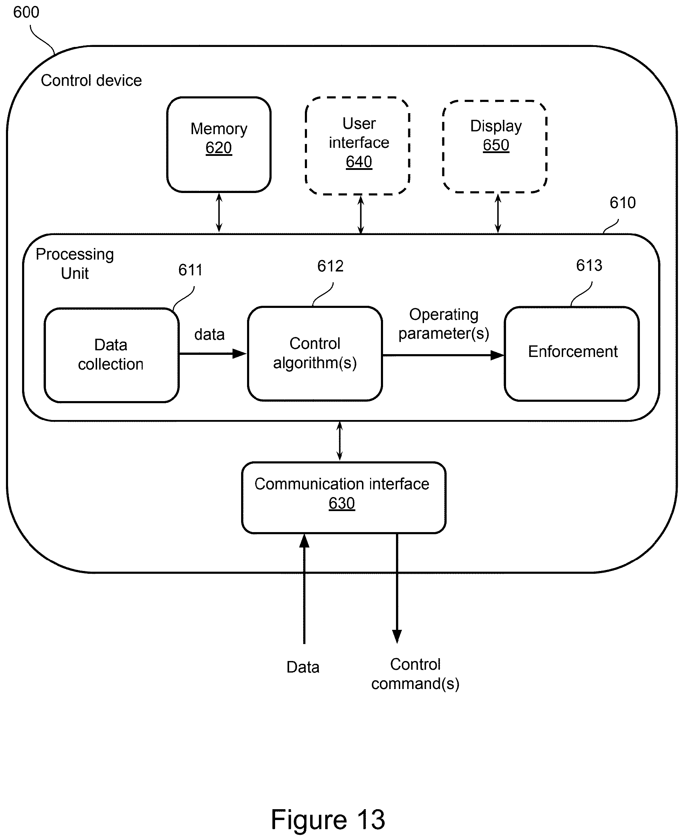

The control device is adapted for initiating and stopping the injection of the raw biogas with hydrogen sulfide in the container 210; and for initiating and stopping the injection of the regeneration gas in the container 210. An exemplary implementation of the control device will be provided later in the description, in relation to Figures 12 and 13.

Optionally, a sensor 225 is used for estimating the quantity (e.g. weight or volume) of material 220 present in the container 210. For example, the sensor 225 is integrated to the container 210 and is capable of directly measuring the quantity (e.g. weight or volume) of material 220 present in the container 210. The measurement performed by the sensor 225 is transmitted to the aforementioned control device. Upon determination by the control device that the quantity of material 220 present in the container 210 is below a predetermined threshold (e.g. only a predefined percentage of the quantity of original material 220 is left), an action is taken (e.g. send a message to a person in charge that some pure material 220 needs to be added to the container 210).

In an exemplary implementation, the sensor 125 is a level sensor. The level sensor is mounted inside and at the top of the container 210, where it emits an ultrasonic signal and receives a diffracted signal back. In addition, the level sensor needs to be isolated during the regeneration mode, to avoid heat damages caused by the hot regeneration gas.

Alternatively or complementarily, the sensor 225 is capable of measuring the quantity (e.g. weight or volume) of one or more by-products extracted from the container 210 (due to the injection of the heated regeneration gas), such as the aforementioned sulfur compound(s), but also water, siloxane compound(s), etc. The sensor 225 may be integrated to the recycling unit 240 as illustrated in Figure 4, but may also be located elsewhere. The measurement performed by the sensor 225 is transmitted to the aforementioned control device. The quantity of by-product measured by the sensor 225 is correlated to the quantity of material 220 present in the container 210. Thus, by measuring the quantity of by-product, an estimation of the quantity of material 220 present in the container 210 is made, and compared to the aforementioned threshold (for taking the aforementioned action). The usage of sensor(s) 225 is not limited to the determination of the quantity of material 220 present (remaining) in the container 210.

More generally, the sensor(s) 225 when positioned downstream of the container 210 is (are) capable of measuring an output flow rate of sulfur compound(s) (e.g. hydrogen sulfide), a concentration of hydrogen sulfide in the regeneration gas, a quantity (e.g. weight) of solid sulfur extracted from the container 210 (by transforming the sulfur compound(s) into solid sulfur, e.g. in the recycling unit 240), a concentration of exhaust gas (e.g. carbon monoxide, sulfur oxide, nitrogen oxide, nitrogen, oxygen, water vapor, etc.), a quantity of oxygen (e.g. percentage or concentration of oxygen) present in the regeneration gas, etc.

Alternatively or complementarily, sensor(s) 225 is (are) positioned upstream of the container 210 and capable of measuring a current input flow rate of the regeneration gas, a quantity of oxygen and / or nitrogen (e.g. percentage or concentration of oxygen and nitrogen) present in the regeneration gas, a temperature of the regeneration gas, etc.

Other types of sensors 225 which can be located inside the container 210 include a temperature sensor for measuring the temperature inside the container 210, a pressure sensor for measuring the pressure inside the container 210, a humidity sensor for measuring the humidity inside the container 210, etc.

Referring back to **Figure 3****,** one or more sensor devices 225 (positioned upstream or downstream of the container 210) can be used in desulfurization mode, for performing one or more of the following measurements: current input flow rate of raw biogas with hydrogen sulfide, current concentration in hydrogen sulfide of the raw biogas entering the container 210, current concentration in hydrogen sulfide of the biogas exiting the container 210, current output flow rate of biogas without hydrogen sulfide, a quantity (e.g. concentration) of oxygen and / or ammonia present in the raw biogas entering the container 210, concentration of exhaust gas extracted from the container 210 (e.g. carbon monoxide, sulfur oxide, nitrogen oxide, nitrogen, oxygen, water vapor, etc.), etc.

The various measurements performed by the various types of sensor devices 225 are used as inputs of one or more algorithms used for controlling the operations of the regenerative hydrogen sulfide capture system. More details about the control algorithm(s) will be provided later in the description.

The implementation of the sensor 225 is out of the scope of the present disclosure and may vary based on the type of measurement to perform (e.g. weight, volume, flow rate, concentration, etc.), the type of product monitored by the sensor 225 (e.g. gas, solid, liquid, etc.), etc. Furthermore, the terminology sensor is used broadly, to encompass any type of equipment capable of performing a measurement that can be used as input of the aforementioned control algorithm(s).

One aspect to be considered is that the material 220 adsorbs water during the capture of hydrogen sulfide. Eliminating the water adsorbed by the material 220 before performing the regeneration of the material 220 with the heated regeneration gas, can help reduce the concentration of carbon monoxide in the flue gas extracted from the container 210 during the regeneration mode. Thus, a drying stage (e.g. 105 degrees Celsius for 6 to 12 hours) can be added to the regeneration process.

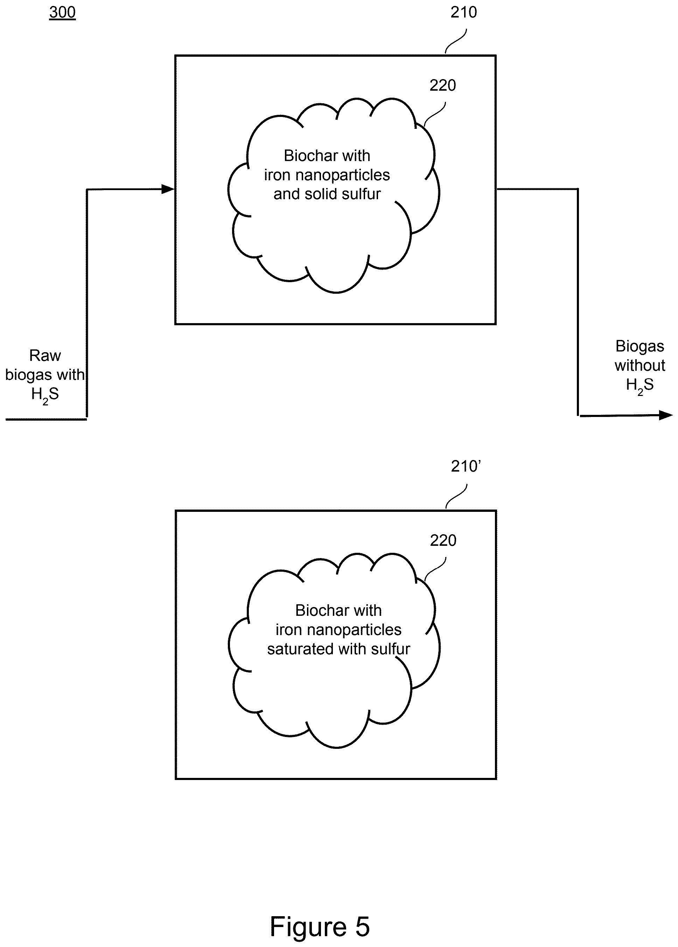

Referring now concurrently to **Figures 5****,** **6****,** **7** **and** **8****,** another implementation of regenerative hydrogen sulfide capture system 300 for removing hydrogen sulfide contained in raw biogas is represented in Figures 5-6 and 7-8. The system 300 is similar to the system 200 illustrated in Figures 2-4, except for operating two containers 210 and 210' in parallel.

Figure 5 illustrates the first container 210 operating as illustrated in Figure 3. Raw biogas containing hydrogen sulfide is injected in the container 210, the hydrogen sulfide is captured and converted into solid sulfur by the material 220, and biogas without hydrogen sulfide is extracted from container 210.

The second container 210' does not receive raw biogas, since the material 220 has been saturated with sulfur (by previously operating as in Figure 3).

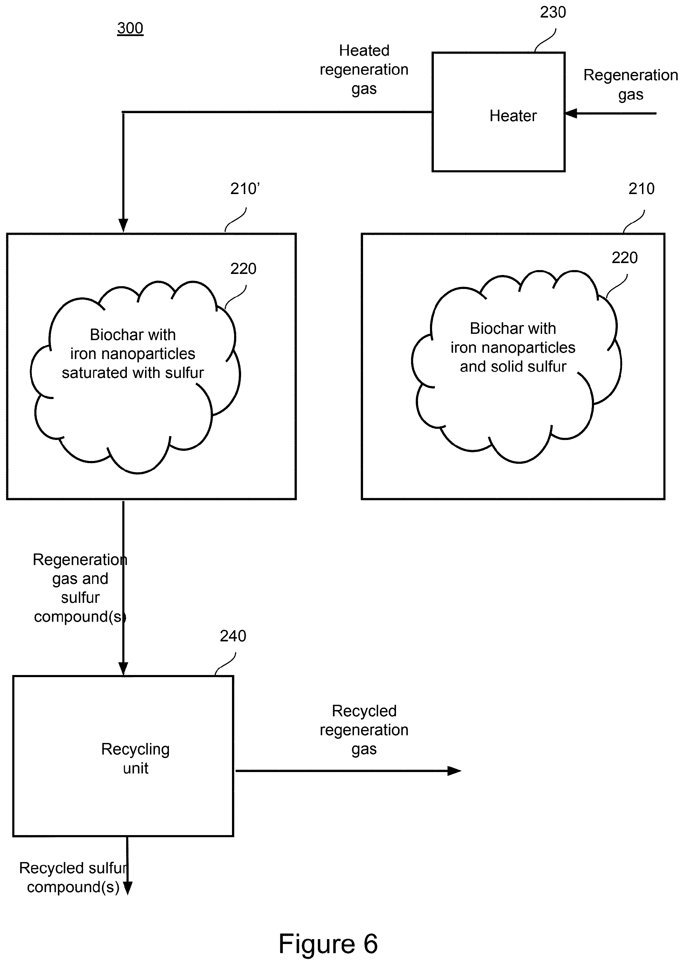

Figure 6 illustrates the second container 210' operating as illustrated in Figure 4. The regeneration gas at the predetermined temperature is injected in the container 210', the (solid) sulfur is extracted from the material 220 in the form of sulfur compound(s). The sulfur compound(s) and the regeneration gas are extracted from the container 210' and processed by the recycling unit 240.

For clarification purposes, the operations of the first container 210 are illustrated in Figure 5 (capture and conversion into solid sulfur of the hydrogen sulfide by the material 220) but not in Figure 6. Similarly, the operations of the second container 210' are illustrated in Figure 6 (regeneration of the material 220) but not in Figure 5. However, the operations illustrated in Figures 5 and 6 are occurring simultaneously.

After a first duration d₁, the material 220 in the second container 210' is regenerated (the material 220 only contains a residual quantity of solid sulfur), as illustrated in Figure 2. At this point, the injection of the regeneration gas is interrupted. Optionally, the container 210' is reloaded with pure material 220 if the maximum number of cycles before reloading is reached.

After a second duration d₂, the material 220 in the first container 210 becomes saturated with sulfur. At this point, the injection of the raw biogas is interrupted.

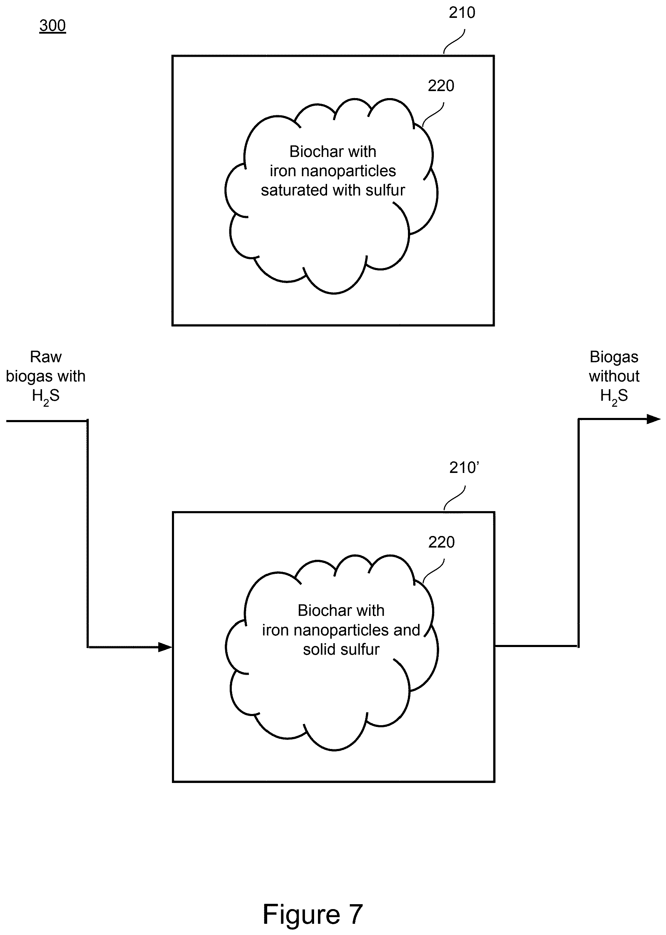

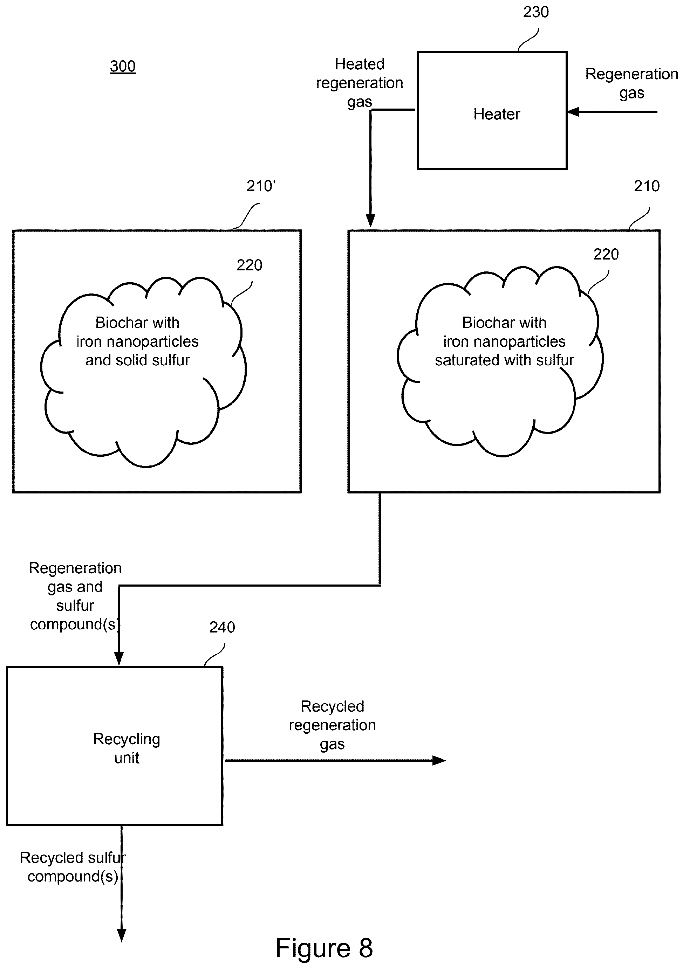

After the longest of d₁ and d₂ duration, the roles of the containers 210 and 210' are inverted, as illustrated in Figures 7 and 8.

Figure 7 illustrates the second container 210' operating as illustrated in Figure 3. Raw biogas containing hydrogen sulfide is injected in the container 210', the hydrogen sulfide is captured and converted into solid sulfur by the material 220, and biogas without hydrogen sulfide is extracted from container 210'.

The first container 210 does not receive raw biogas, since the material 220 has been saturated with sulfur (by previously operating as in Figure 3).

Figure 8 illustrates the first container 210 operating as illustrated in Figure 4. The regeneration gas at the predetermined temperature is injected in the container 210, the (solid) sulfur is extracted from the material 220 in the form of sulfur compound(s). The sulfur compound(s) and the regeneration gas are extracted from the container 210 and processed by the recycling unit 240.

As mentioned previously, for clarification purposes, the operations of the second container 210' are illustrated in Figure 7 (capture and conversion into solid sulfur of hydrogen sulfide by the material 220) but not in Figure 8. Similarly, the operations of the first container 210 are illustrated in Figure 8 (regeneration of the material 220) but not in Figure 7. However, the operations illustrated in Figures 7 and 8 are occurring simultaneously.

The advantage of the system 300 is that by alternating the operations of the container 210 and 210' (hydrogen sulfide removal and regeneration of material 220), the removal of hydrogen sulfide from biogas is a continuous process which does not need to be interrupted (or at least for a significantly lower amount of time in comparison to the system 200 illustrated in Figures 2-4).

As mentioned previously (with respect to the system 200 illustrated in Figures 2-4), a control device is used for synchronizing the operations of the containers 210 and 210'. In particular, the control device controls the transitions between the two modes of operation (hydrogen sulfide removal and regeneration of material 220) for each of the containers 210 and 210'.

In an exemplary implementation, the control device sends control commands to a plurality of valves controlling the inputs and the outputs of the containers 210 and 210'. The valves are not represented in Figures 5-9 for simplification purposes. Each container 210 and 210' is connected to the source of raw biogas through an input valve. Each container 210 and 210' is connected to the equipment receiving the biogas without hydrogen sulfide through an output valve. In the configuration illustrated in Figure 5, the input and output valves of container 210 are opened, while the input and output valves of container 210' are closed. In the configuration illustrated in Figure 7, the input and output valves of container 210 are closed, while the input and output valves of container 210' are opened.

Similarly, each container 210 and 210' is connected to the heater 230 through an input valve, which controls injection of the heated regeneration gas in each respective container 210 and 210'. Similarly, each container 210 and 210' is connected to the recycling unit 240 through one or more output valves, which control extraction of the regeneration gas and sulfur compound(s) from each respective container 210 and 210'.

In another exemplary implementation, each container 210 and 210' has four valves to operate in the two different modes. Two biogas valves are connected to a biogas line supplying raw biogas during the desulfurization mode. Two regeneration gas valves are connected to a regeneration gas line supplying regeneration gas during the regeneration mode. Once the material 220 is saturated with sulfur (one of the previously described methods is used for detecting the saturation), the biogas line is closed (by closing the two biogas valves) and the container 210 (or 210') is no longer supplied with raw biogas. The regeneration gas line is opened (by opening the two regeneration gas valves) and the container 210 (or 210') is supplied with regeneration gas, to operate in regeneration mode. Once the regeneration mode is completed, the two regeneration gas valves are closed and the two biogas valves are reopened. The container 210 (or 210') and regenerated material 220 are ready for another cycle of performing the desulfurization mode followed by the regeneration mode.

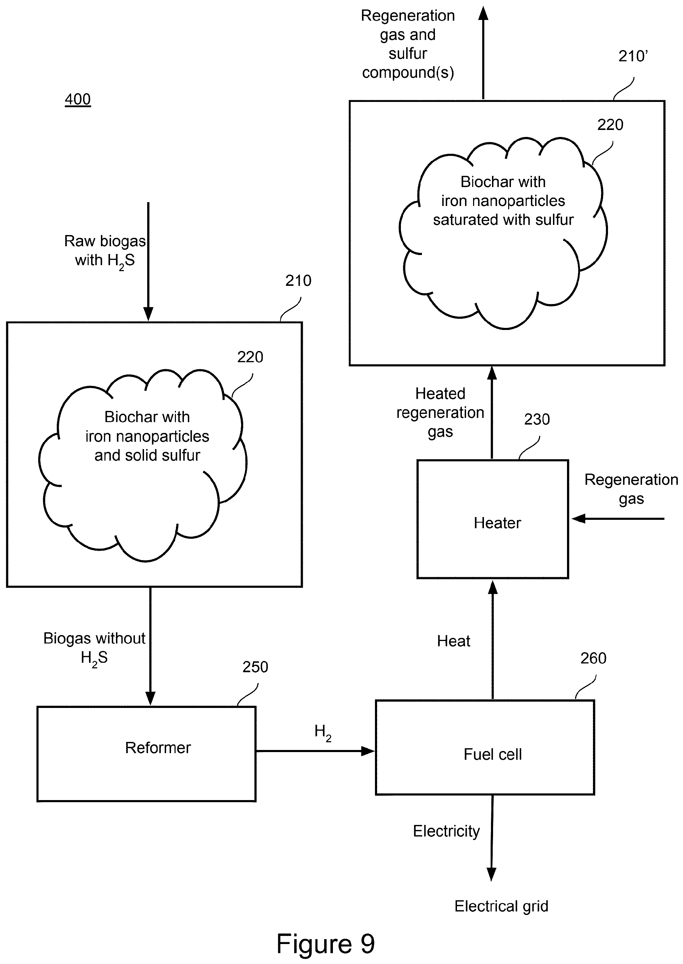

Referring now to Figure 9, another implementation of a regenerative hydrogen sulfide capture system 400 for removing hydrogen sulfide contained in raw biogas and further generating electricity is represented. The system 400 is similar to the system 300 illustrated in Figures 5-6, with the addition of a reformer 250 and a fuel cell 260.

A fuel cell is well known in the art. The fuel cell 260 uses hydrogen (H₂) and oxygen (O₂) to generate electricity (in reverse electrolysis mode). A by-product of the fuel cell is water (H₂O), generated by the chemical reaction between hydrogen and oxygen to generate electricity. Another by-product is heat generated by the chemical reaction. The electricity generated by the fuel cell 260 can be injected into the electrical grid (as illustrated in Figure 9) and / or used locally for powering local equipment.

Raw biogas containing hydrogen sulfide is injected in the container 210, the hydrogen sulfide is captured and converted into solid sulfur by the material 220, and biogas without hydrogen sulfide is extracted from container 210. The biogas without hydrogen sulfide is injected in the reformer 250. Operations of a reformer are also well known in the art. The reformer 250 processes the biogas to produce the hydrogen injected in the fuel cell 260.

The heat generated by the fuel cell 260 is used by the heater 230 for heating the regeneration gas at the predetermined temperature. The regeneration gas at the predetermined temperature is injected in the container 210', the (solid) sulfur is extracted from the material 220 in the form of sulfur compound(s). The sulfur compound(s) and the regeneration gas are extracted from the container 210' and processed by the recycling unit 240 (illustrated in Figure 6 but not represented in Figure 9 for simplification purposes).

Although not represented in the Figures for simplification purposes, the roles of the containers 210 and 210' with respect to the fuel cell 260 are inverted after a while.

More specifically, referring to Figures 7 and 8, the container 210' contains (initially) purified material 220 used for removing hydrogen sulfide from biogas to produce biogas without hydrogen sulfide, which is used to feed the fuel cell 260 through the reformer 250. The regeneration gas heated at the predetermined temperature through the heat generated by the fuel cell 260 is injected in the container 210, to purify the material 220 which has been saturated with sulfur.

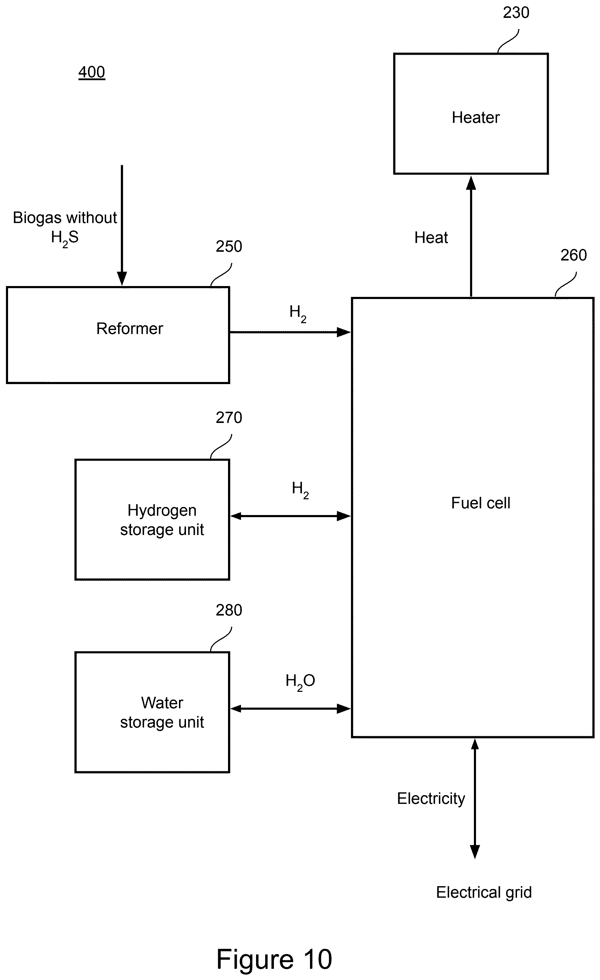

Referring now concurrently to Figures 9 and 10, Figure 10 illustrates the fuel cell 260 of Figure 9 being adapted to also operate in electrolysis mode. Some of the components of the regenerative hydrogen sulfide capture system 400 of Figure 9 are not represented in Figure 10 for simplification purposes only.

When demand for electricity is high in the electrical grid (and consequently the price of electricity may be higher), the fuel cell 260 operates in reverse electrolysis mode (as previously illustrated in Figure 9), using the hydrogen generated by the reformer 250 and oxygen to produce electricity (injected in the electrical grid and / or used locally). The heat generated as a byproduct is used by the heater 230. The water generated as a byproduct can be stored in a water storage unit 280.

When demand for electricity is low in the electrical grid (and consequently the price of electricity may be lower), the fuel cell 260 operates in electrolysis mode. Electricity from the electrical grid is used to electrolyze water stored in the water storage unit 280. to produce hydrogen and oxygen. The produced hydrogen is stored in a hydrogen storage unit 270. Heat is also generated as a byproduct of the electrolysis, which can be used by the heater 230 or another equipment on the premises.

When demand for electricity becomes high again in the electrical grid, the hydrogen stored in the hydrogen storage unit 270 is used to operate the fuel cell 260 in reverse electrolysis mode, as described previously.

As will be detailed later in relation to Figure 12, a combination of hardware and software is used to control operations of the fuel cell 260. For example, to determine when the fuel 260 is operating in reverse electrolysis or electrolysis modes, based on the electricity demand in the electrical grid. Also, when hydrogen is available simultaneously from the reformer 250 and the hydrogen storage unit 270, to determine which source of hydrogen is used (the reformer 250 only, the hydrogen storage unit 270 only, or a combination of the two) to operate in reverse electrolysis mode to produce electricity. Optionally, the control of the operations of the fuel cell 260 also takes into consideration biogas forming reactions, which yield carbon monoxide that can be used as fuel gas in a fuel cell to produce electricity.

In an alternative configuration, water is not stored in the water storage unit 280 in electrolysis mode, but simply disposed of. Another source of water (not represented in the Figures) is used for providing the water needed to operate in reverse electrolysis mode.

In another alternative or complementary configuration, at least some of the hydrogen generated when operating the fuel 260 in electrolysis mode is not used for later operating the fuel 260 in reverse electrolysis mode, but is used (in combination with carbon dioxide) to further produce methane. The produced methane can be stored in the gasometer 150 illustrated in Figure 1 or in another storage unit, and used later by the reformer 250 to produce hydrogen for operating the fuel cell 260 in reverse electrolysis mode (or used for another purpose). Some of the hydrogen generated by the reformer 250 can also be used to produce methane (for example, if the reformer 250 produces more hydrogen than is needed for operating the fuel cell 260 in reverse electrolysis mode). Similarly, at least some of the hydrogen generated by the gasometer 150 or by the fuel cell 260 (when operating in reverse electrolysis mode) can be injected in the digester 120 of Figure 1, to increase the yield of raw biogas generation by the digester 120.

Furthermore, source(s) of methane available on the premises can also be used by the reformer 250 to produce hydrogen for the fuel cell 260 (in addition to the aforementioned biogas). For example, in the context of a cattle farm, methane is accumulated in the barn(s), which can be captured and injected in the reformer 250.

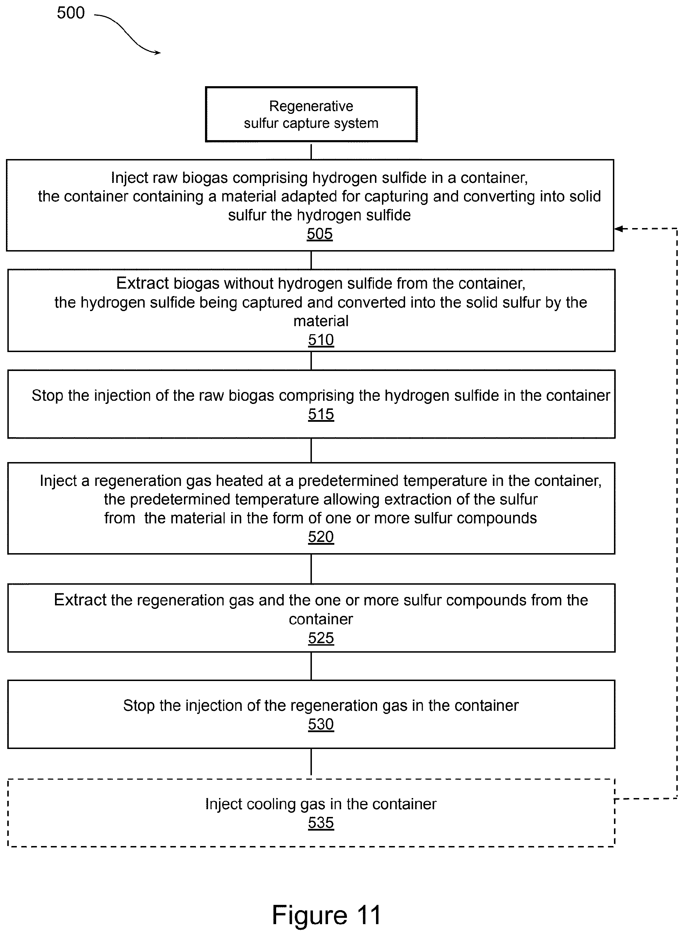

Referring now concurrently to **Figures 1****,** **2****,** **3****,** **4****,** **5****,** **6****,** **7****,** **8****,** **9** **and** **11****,** a method 500 for performing a regenerative desulfurization procedure of raw biogas is represented in Figure 11.

The following steps of the method 500 are performed by the system 200 illustrated in Figures 2-4.

The method 500 comprises the step 505 of injecting the raw biogas comprising the hydrogen sulfide in the container 210, the container 210 containing the material 220 adapted for capturing and converting into solid sulfur the hydrogen sulfide.

The method 500 comprises the step 510 of extracting biogas without hydrogen sulfide from the container 210, the hydrogen sulfide being captured and converted into the solid sulfur by the material 220.

The method 500 comprises the step 515 of stopping the injection of the raw biogas comprising the hydrogen sulfide in the container 210.

The method 500 comprises the step 520 of injecting the regeneration gas heated at the predetermined temperature in the container 210. The predetermined temperature allows extraction of the (solid) sulfur from the material 220 in the form of sulfur compound(s).

The method 500 comprises the step 525 of extracting the regeneration gas and the sulfur compound(s) from the container 310.

The method 500 comprises the step 530 of stopping the injection of the regeneration gas in the container 310.

The method 500 comprises the optional step 535 of injecting a cooling gas in the container 310, to cool the heated material 220. The cooling gas is the regeneration gas injected at a lower temperature or another gas.

Optionally, steps 505-530 are repeated several times. As mentioned previously, after several repetitions of steps 505-530 (e.g. after 6 repetitions), a quantity of pure material 220 is added to the container 210, to compensate for the loss of material 220. After the addition of pure material 220, steps 505-530 are performed again. The step of adding pure material 220 is not represented in Figure 11 for simplification purposes.

The step of adding pure material can be implemented as follows. Initially, a cooling phase is needed, to cool down the content of the container 210 (control devices for the protection of people, such as a light indicating the temperature and automatically closing doors, can be used during the cooling phase). During the cooling phase, the container 210 is filled with the regeneration gas (nitrogen and oxygen). Subsequently, when the temperature is sufficiently low, the container 210 is isolated from the raw biogas, by sending nitrogen and oxygen (nontoxic) under pressure in the container 210 (the rise of pressure isolates the container 210). The container 210 is adapted to allow opening of a hatch or an opening at the top (to push the pure material in the container 210 from the top), or to actuate a vacuum valve to transfer the pure material towards the container 210. Once the container 210 is filled with the appropriate quantity of material, the container 210 is closed manually or by the vacuum valve (a test of pressure can be used to ensure that the container 210 is properly closed). Then, a purge of nitrogen and oxygen is performed to remove the air in the container 210. Subsequently, the container 210 is reconnected to the source of raw biogas, so that the mixture of nitrogen and oxygen is sent gradually towards the rest of the system without exceeding permitted quantities in the rest of the system. The concentration of oxygen in the gas exiting the container 210 needs to be monitored, to ensure that the proportion (e.g. concentration or percentage) of oxygen does not exceed a threshold (e.g. a maximum proportion which may vary). The method 500 is also applicable to the system 300 illustrated in Figures 5-6 and 7-8. More specifically, the method 500 is implemented simultaneously on containers 210 and 210', with the following synchronization constraints.

Steps 505 and 510 are applied to container 210 while steps 520 and 525 are applied simultaneously to container 210'.

Steps 520 and 525 are applied to container 210 while steps 505 and 510 are applied simultaneously to container 210'.

Referring now concurrently to **Figures 1****,** **2****,** **4, 4****,** **5****,** **6****,** **7****,** **8****,** **9****,** **10** **and** **12****,** a control device 600 is represented in Figure 12. The control device 600 is used for controlling operations of components of the systems 200 (illustrated in Figures 2-4), 300 (illustrated in Figures 5-6 and 7-8) and 400 (illustrated in Figures 9 and 10), and optionally of components of the system 100 (illustrated in Figure 1). For simplifications purposes, only the components interacting with the control device 600 are represented in Figure 12.

The control device 600 implements three functionalities: data collection 611, one or more control algorithms 612 and enforcement 613. Each functionality is performed by executing instructions of one or more computer programs. Hardware components of the control device 600 will be detailed later in the description, including a processing unit capable of executing the instructions of the one or more computer programs.

The data collection functionality 611 collects data from one or more entities, including at least one of the following: the container 210, the second container 210' if present, the heater 230, the recycling unit 240, the digester 120, the gasometer 150 if present, the energy generator 160 (e.g. the reformer 250 and the fuel cell 260, the electrical grid), etc.

Following are examples of the collected data. The following examples are for illustration purposes only. A person skilled in the art would readily understand that other types of data may be collected. The collected data are representative of operating conditions (current and / or future) of components of the regenerative hydrogen sulfide capture systems 200, 300 and 400.

A first exemplary type of collected data comprises operating condition(s) of the container 210 (and / or 210', as illustrated in Figures 2-9). The operating condition(s) of each container can be collected in different ways, including direct transmission by a component of the container, transmission by a sensor monitoring the operations of the container, etc.

Examples of operating condition(s) of the container 210 (and 210' if present) comprise current operating mode (hydrogen sulfide capture by material 220 or regeneration of material 220), current input flow rate of raw biogas with hydrogen sulfide in desulfurization mode, current concentration in hydrogen sulfide of the raw biogas entering the container 210 (and 210' if present) in desulfurization mode, current concentration in hydrogen sulfide of the biogas exiting the container 210 (and 210' if present) in desulfurization mode (to determine when to start the regeneration mode, e.g. when the concentration falls below a threshold representative of the material 220 being saturated with sulfur), current output flow rate of biogas without hydrogen sulfide in desulfurization mode, estimation of a current concentration of sulfur in the material 220 (also referred to as a current quantity of sulfur absorbed by the material 220) in desulfurization mode (an increase of the concentration of sulfur is representative of the material 220 becoming more and more saturated with sulfur), current input flow rate of regeneration gas in regeneration mode, current output flow rate of sulfur compound(s) in regeneration mode (an increase of the output flow rate of sulfur compound(s) is representative of the material 220 being regenerated, a decrease of the output flow rate of sulfur compound(s) is representative of the material 220 reaching complete regeneration), estimation of a current concentration of sulfur in the material 220 in regeneration mode (a decrease of the concentration of sulfur is representative of the material 220 being regenerated), etc.

Additional examples of operating condition(s) comprise current (instant) quantity (e.g. weight) of solid sulfur extracted from the container 210 (and 210' if present) while in regeneration mode (to determine when the regeneration mode is completed, which corresponds to (substantially) no more sulfur being extracted from the material 220, and consequently form the container), total quantity (e.g. weight) of solid sulfur extracted from the container 210 (and 210' if present) at the end of the regeneration mode (to predict the quantity of material 220 remaining in the container - the adsorption capacity of the material 220 is known and is a given weight percent loading of sulfur, recovering a lower total quantity of solid sulfur suggests that there is less material 220 inside the container), quantity of oxygen and / or ammonia (e.g. concentration or percentage) present in the raw biogas in desulfurization mode (the oxygen and ammonia facilitate the capture of sulfur by the material 220, and the quantity of oxygen and / or ammonia can be used to predict when the saturation in sulfur of the material 220 of occurs), the temperature of the regeneration gas in regeneration mode (e.g the target is 600 degrees Celsius but variations need to be monitored), temperature inside the container 210 (and 210' if present) which can be measured by a temperature sensor installed therein, pressure inside the container 210 (and 210' if present) which can be measured a pressure sensor installed therein, humidity inside the container 210 (and 210' if present) which can be measured a humidity sensor installed therein (a too high level of humidity can damage the material 220) (a knock-out tank positioned before the container 210 (and 210' if present) can be used to reduce the humidity level of the biogas and / or regeneration gas injected in in the container 210 (and 210' if present), quantity of oxygen and nitrogen (e.g. concentration or percentage of oxygen and nitrogen) present in the regeneration gas in regeneration mode (this quantity is supposed to remain constant and a change may have an impact on the quantity (mass) of material 220 lost during the regeneration mode), comparison of the quantity of oxygen (contained in the regeneration gas) injected in and extracted from the container 210 (and 210' if present) in regeneration mode (to predict the loss of mass of the material 220 - a result of the comparison being substantially zero means that all the oxygen injected in the container has reacted with the material 220 (nominal loss of material mass), while a result of the comparison being greater than zero means that some of the oxygen injected in the container has not reacted with the material 220 (lower loss of material mass)), concentration of exhaust gas (extracted from the container 210 (and 210' if present) during the desulfurization mode and / or the regeneration mode (e.g. carbon monoxide, sulfur oxide, nitrogen oxide, nitrogen, oxygen, water vapor, etc.).

Optionally, weather conditions are also taken into account for controlling the operations of the container 210 (and 210' if present). The current external temperature and predictions of future external temperature, can be used to determine when to start the regeneration mode. The regeneration mode (which can for example take two days to complete) does not need to start immediately after the desulfurization mode, but can be delayed for several days, to wait for optimal weather conditions (e.g. sunny, without snowstorm or thunderstorm, warm and dry). Furthermore, picking optimal weather conditions can reduce the electrical costs of operating the system, by avoiding periods of higher price for electricity (which can be tied to weather conditions, such as extreme cold or warm conditions).

A second exemplary type of collected data comprises operating condition(s) of the digester 120 (illustrated in Figure 1). The operating condition(s) of the digester 120 can be collected in different ways, including direct transmission by a component of the digester 120, transmission by a sensor monitoring the operations of the digester 120, etc.

Examples of operating condition(s) of the digester 120 comprise an organic loading rate of the digester 120, a quantity of manure contained in the digester 120, a composition of the content of the digester 120 (e.g. proportion of gas versus solid content, a quantity of water input), a concentration and / or dosage of iron chloride in the digester 120, a temperature of the digester 120, the addition of an external feedstock in the digester 120, the type of additional feedstack (which influences the concentration of hydrogen sulfide in the raw biogas), biogas catalyst (used to increase the raw biogas production once added), etc.

Furthermore, the source of the raw biogas is not limited to a digester, but may also include a landfill, a water treatment unit, etc. Thus, depending on the source of raw biogas, information (e.g. concentration) related to the gases (e.g. oxygen, ammoniac, hydrogen sulfide, methane, carbon dioxide, humidity, etc.) contained in the raw biogas produced by the source can be collected and used by the control algorithm(s) 612. This information is relevant to the quality of adsorption of the material 220 and the frequency of the regeneration phase. Thus, the information can be used by the control algorithm(s) 612 to predict the frequency of the regeneration phase, the number of regeneration phases occurring in a period of time (e.g. one year), etc.

A third exemplary type of collected data comprises operating condition(s) of the fuel cell 260 (illustrated in Figures 9 and 10). For example, data representative of the level of demand for electricity in the electrical grid are collected. As mentioned previously, a higher demand for electricity may result in higher prices for consumed electricity while a lower demand for electricity may result in lower prices for consumed electricity. Another example of operating conditions of the fuel cell 260 comprises the determination of whether hydrogen is available from the reformer 250 and the hydrogen storage unit 270 (in order to operate the fuel cell 260 in reverse electrolysis mode).

The one or more control algorithms 612 process the data collected by the data collection functionality 611, to generate operating parameter(s).

The inputs of a given control algorithm 612 comprise a combination of information belonging to any of the previously mentioned exemplary types of collected data.

The output of a given control algorithm 612 comprises one or more operating parameters applicable to one or more entities of the previously described regenerative hydrogen sulfide capture systems (100, 200, 300 or 400). More specifically, each operating parameter is applicable to one of the following entities: the container 210, the second container 210' if present, the digester 120, the heater 230, the recycling unit 240, the gasometer 150 if present, the energy generator 160 (e.g. the reformer 250 and the fuel cell 260), etc.

The control algorithm 612 is designed to determine optimized values for the operating parameters based on the inputs, in order to reach a goal. An exemplary goal for the systems 300 and 400 is to continuously process the raw biogas containing hydrogen sulfide (without interruptions), by optimally managing operations of the containers 210 and 210'. For this purpose, the control algorithm 612 synchronizes the modes of operation of the containers 210 and 210', so that one container is operating in desulfurization mode while the other is operating in regeneration mode. Furthermore, the occurrence of the transition from one mode to the other may also be optimized, to preserve the integrity of the material 220, to improve the efficiency of the whole process, etc. For example, the transition from the desulfurization mode to the regeneration mode is triggered when a predetermined level of saturation of the material 220 with sulfur (measured by a sensor) is reached. In another example, the transition from the desulfurization mode to the regeneration mode is triggered when a concentration of hydrogen sulfide in the biogas exiting the tank operating in desulfurization mode (measured by a sensor) increases up to a threshold representative of a saturation of the material 220 with sulfur (the raw biogas entering the tank can no longer be adequately purified from the hydrogen sulfide). Additionally, a transition from the regeneration mode to an idle mode can be triggered once the regeneration is completed. The container stays in idle mode for a period of time, before entering the desulfurization mode again. While in idle mode, pure material 220 can be loaded in the container to compensate for the loss of material 220.

Another exemplary goal for the systems 300 and 400 is to accurately and reliably determine when to transition from the desulfurization mode to the regeneration mode. During the desulfurization mode, data representative of the current operating condition are collected to plan (and adjust in real-time) when the saturation will occur based on the collected data. The prediction is performed by algorithm(s) taking into account the previously collected data and the currently collected data. For example, the prediction is based on the measure of the concentration of hydrogen sulfide in the raw biogas injected in the container 210 over a period of time. During this period of time, a total quantity X of hydrogen sulfide has been injected and captured by the material 220. Based on the total quantity X of captured sulfur, it can be determined algorithmically a percentage Y of saturation in sulfur of the material 220. Based on the evolution of the concentration of hydrogen sulfide over time and the current percentage Y of saturation, it can be predicted algorithmically when (e.g. a time window) the level of saturation will reach a level that triggers the regeneration mode. Furthermore, the algorithm can also be used to predict when pure material 220 needs to be available (for example, immediately after the regeneration mode has been performed, pure material 220 needs to be added to compensate for the loss in weight of the material 220 during the regeneration mode). Furthermore, predicting the time window when container 210 needs to operate in regeneration mode also predicts when container 210' needs to be ready to operate in desulfurization mode (and to take appropriate measures if for some reasons it is expected that container 210' will not be ready to operate in desulfurization mode).

The control algorithm 612 can be implemented by a standard algorithm or a machine learning algorithm (e.g. a neural network). In the case of a machine learning algorithm, historical training data are used to train the control algorithm 612, so that current values of the inputs of the control algorithm 612 result in corresponding optimized values for the operating parameters based on the training (e.g. determination of the weights of a neural network during the training phase using the historical training data).

The enforcement functionality 613 applies each operating parameter generated by the control algorithm(s) 612 to one one of the previously mentioned entities to which the operating parameter is applicable. In an exemplary implementation, the enforcement functionality 613 generates control command(s) for enforcing the operating parameter(s) generated by the control algorithm(s) 612. Each control command is transmitted to one of the previously mentioned entities. For simplification purposes only, Figure 12 represents control commands being sent to container 210 (and 210' if present). For example, a control command for enforcing one or more operating parameters of the container 210 is transmitted to the container 210, a control command for enforcing one or more operating parameters of the container 210' (if present) is transmitted to the container 210', etc.

A first exemplary type of operating parameters comprises operating parameters of the container 210 (and 210' if present). Examples of operating parameters of the container 210 (and 210' if present) comprise when a mode of operation (desulfurization mode, regeneration mode, idle) needs to be initiated or stopped, initiating or stopping the injection of raw biogas with hydrogen sulfide in the container, a flow rate of raw biogas with hydrogen sulfide injected in the container, initiating or stopping the extraction of biogas without hydrogen sulfide from the container, a flow rate of biogas without hydrogen sulfide extracted from the container, initiating or stopping the injection of heated regeneration gas in the container, a flow rate of heated regeneration gas injected in the container, initiating or stopping the extraction the regeneration gas with sulfur compound(s) from the container, a flow rate of the regeneration gas with sulfur compound(s) extracted from the container, etc. Other examples of operating parameters which can be determined by the control algorithm(s) 612 have been mentioned previously, when describing the various types of operating conditions which can be used as inputs of the control algorithm(s) 612.

The enforcement of an operating parameter by an entity (e.g. container 210 or container 210' if present) of the regenerative hydrogen sulfide capture systems is well known in the art. In an exemplary implementation, the entity comprises a communication interface for receiving the control command sent by the control device 600 to enforce the operating parameter. The entity generally comprises a processing unit for processing the control command to activate one or more actuators of the entity, the one or more actuators enforcing the operating parameter. For example, referring to the previously mentioned input and output valves of the containers 210 (and 210' if present), the controlled entity is a local control device associated to one or more valves, the local control device receiving the control commands from the central control device 600 and enforcing the received control commands by opening or closing the valve(s) under its control according to the received control commands.

A second exemplary type of operating parameters comprises operating parameters of the fuel cell 260 (represented in Figure 10). Examples of operating parameters of the fuel cell 260 comprise mode of operation (reverse electrolysis mode or electrolysis mode), source of hydrogen (reformer 250, hydrogen storage unit 270, or both in respective determined proportions) when operating in reverse electrolysis mode, etc. A detailed implementation of the enforcement of the aforementioned operating parameters is beyond the scope of the present disclosure. As mentioned previously, control commands are sent by the enforcement functionality 613 to one or more entities in charge of controlling at least one of the fuel cell 260, reformer 250, hydrogen storage unit 270, etc. (to effectively enforce the operating parameters). The operating parameters are determined by the control algorithm(s) 612 based on the previously mentioned collected data representative of operating conditions of the fuel cell 260.

For example, the control algorithm 612 determines that the fuel cell 260 operates in reverse electrolysis mode when demand for electricity is high and operates in electrolysis mode when demand for electricity is low. In another example, when hydrogen is available from both the hydrogen storage unit 270 and the reformer 250 to operate in reverse electrolysis mode, the control algorithm 612 determines that hydrogen from the hydrogen storage unit 270 is used in priority. When the hydrogen storage unit 270 is emptied, hydrogen from the reformer 250 is used.

Referring now concurrently to **Figures 12** **and** **13****,** a schematic representation of components of the control device 600 is illustrated in Figure 13. The control device 600 may be implemented by different types of computing devices, including a computer, a server, a tablet, a smartphone, an electronic board, etc.

The control device 600 comprises a processing unit 610, memory 620, a communication interface 630, optionally a user interface 640, and optionally a display 650. The control device 600 may comprise additional components not represented in Figure 13 for simplification purposes (e.g. an additional communication interface 630).

The processing unit 610 comprises one or more processors (not represented in Figure 12) capable of executing instructions of computer programs. Each processor may further comprise one or several cores. Alternatively or complementarily, the processing unit 610 comprises a Field Programmable Gate Array (FPGA), an application-specific integrated circuit (ASIC), etc.

The memory 620 stores instructions of computer programs executed by the processing unit 610, data generated by the execution of the computer programs, data received via the communication interface 630, etc. Only a single memory 620 is represented in Figure 12, but the control device 600 may comprise several types of memories, including volatile memory (such as a volatile Random Access Memory (RAM), etc.) and non-volatile memory (such as a hard drive, solid-state drive (SSD), electrically-erasable programmable read-only memory (EEPROM), flash, etc.). Alternatively or complementary, the memory 620 is (at least partially) integrated to the processing unit 610.

The communication interface 630 allows the control device 600 to exchange information with other devices (as illustrated in Figure 11) over one or more communication networks (not represented in Figure 13 for simplification purposes). The term communication interface 630 shall be interpreted broadly, as supporting a single communication standard / technology, or a plurality of communication standards / technologies. Examples of communication interfaces 630 include a wireless (e.g. Wi-Fi, cellular, wireless mesh, etc.) communication module, a wired (e.g. Ethernet) communication module, a combination of wireless and wired communication modules, etc. The communication interface 630 usually comprises a combination of hardware and software executed by the hardware, for implementing the communication functionalities of the communication interface 630.

The data collection functionality 611, the control algorithm(s) 612 and the enforcement functionality 613 are executed by the processing unit 610. The data collected by the data collection functionality 611 are received via the communication interface 630 and the control commands generated by the enforcement functionality 613 are transmitted via the communication interface 630.

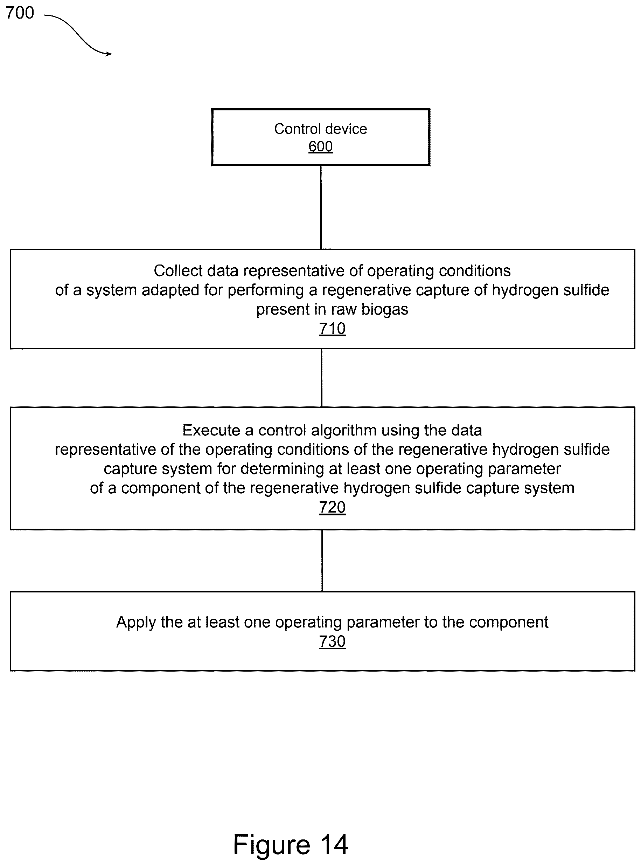

Referring now concurrently to **Figures 12****,** **13** **and** **14****,** a method 700 for controlling a regenerative desulfurization procedure of raw biogas is illustrated in Figure 14. At least some of the steps of the method 700 are executed by the control device 600.

One or more dedicated computer programs have instructions for implementing at least some of the steps of the method 700. The instructions are comprised in a non-transitory computer-readable medium (e.g. the memory 620) of the control device 600. The instructions, when executed by the processing unit 610 of the control device 600, provide for controlling a regenerative capture of hydrogen sulfide present in raw biogas (by implementing the method 700). The instructions are deliverable to the control device 600 via an electronically-readable media such as a storage media (e.g. CD-ROM, or any internally or externally attached storage device connected via USB, Firewire, SATA, etc.), or via communication links (e.g. via a communication network through the communication interface 630).

The method 700 comprises the step 710 of collecting data representative of operating conditions of a system adapted for performing a regenerative capture of hydrogen sulfide present in raw biogas. This step is executed by the processing unit 610 of the control device 600. The data are received via the communication interface 630 of the control device 600. This step is implemented by the data collection functionality 611 and has been detailed previously in relation to Figure 12.

The method 700 comprises the step 720 of executing the control algorithm 612, the control algorithm 612 using the data collected at step 710 for determining at least one operating parameter of a component of the regenerative hydrogen sulfide capture system. This step is executed by the processing unit 610 of the control device 600 and has been detailed previously in relation to Figure 12.

The method 700 comprises the step 730 of applying the at least one operating parameter determined at step 720 to the component (to control the component). In an exemplary implementation, step 730 comprises transmitting one or more control commands to the component for enforcing the at least one operating parameter. The one or more control commands are generated based on operating parameter(s). This step is executed by the processing unit 610 of the control device 600. The one or more control commands are transmitted via the communication interface 630 of the control device 600. This step is implemented by the enforcement functionality 613 and has been detailed previously in relation to Figure 12.

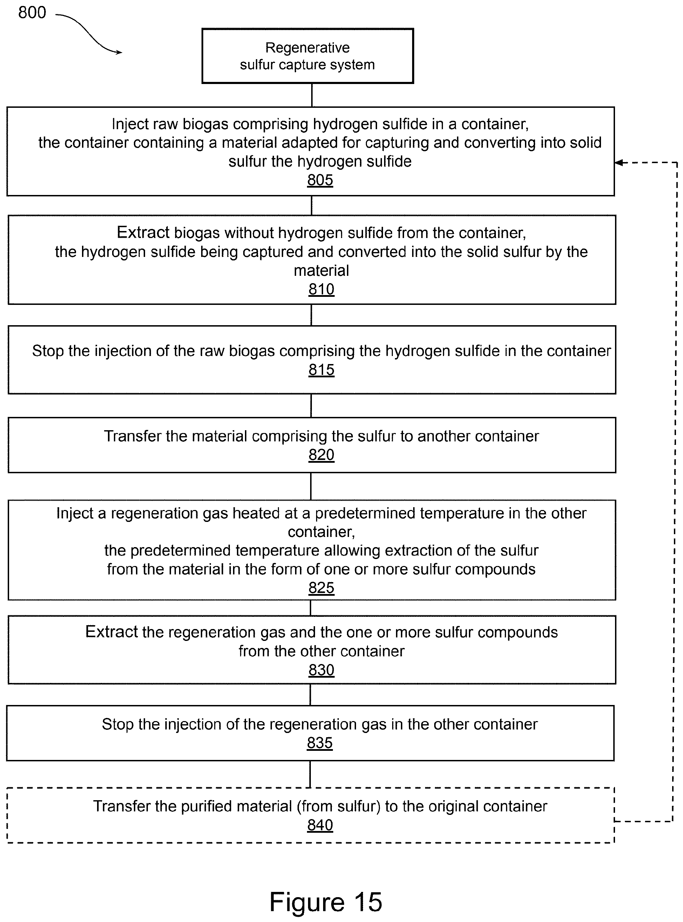

Referring now concurrently to **Figures 5****,** **6****,** **8****,** **11** **and** **15****,** another method 800 for performing a regenerative desulfurization of raw biogas is represented in Figure 15. The method 800 is similar to the method 500 illustrated in Figure 11, with the exception that the desulfurization mode and the regeneration mode are performed with the same container for method 500 (e.g. container 210 illustrated in Figures 5 and 8), while they are performed with two different containers for method 800 (e.g. container 210 illustrated in Figure 5 for the desulfurization mode and container 210' illustrated in Figure 6 for the regeneration mode).

Steps 805, 810 and 815 of the method 800 are similar to respective steps 505, 510 and 515 of the method 500. For example, steps 805-815 are performed with container 210 illustrated in Figure 5.

The method 800 comprises the step 820 of transferring the material 220 comprising the sulfur to another container (than the one that was used for performing steps 805-815). Step 820 may occur anytime after step 815, step 815 occurring when it is determined that the material 220 is saturated with sulfur and can no longer perform the capture of hydrogen sulfide from the raw biogas according to step 810.

The other container is located at the same or different premises than the container used for steps 805-815. Optionally, the other container is a mobile container located on a vehicle (e.g. a truck). The mobile container can be easily moved in close vicinity of any container performing steps 805-815, to proceed with the transfer of the material 220 comprising the sulfur according to step 820.

Steps 825, 830 and 835 of the method 800 are similar to respective steps 520, 525 and 530 of the method 500. The only difference is that steps 835-835 are performed with the other container (than the one used for performing steps 805-815), while steps 520-530 are performed with the same container that was used for performing steps 505-515. For example, steps 825-835 are performed with container 210' illustrated in Figure 6.

The method 800 comprises an optional step performed after step 835, of injecting a cooling gas in the container 310, to cool the heated material 220. This optional step is not represented in Figure 15 for simplification purposes and corresponds to step 535 of the method 500.

The method 800 comprises the optional step 840 of transferring the purified material (from sulfur) 220 to the original container (the one that was used for performing steps 805-815). Step 840 may occur anytime after step 835, step 835 occurring when it is determined that the material 220 has been sufficiently purified according to step 825. After the transfer, a new cycle of steps 805-835 can occur, as illustrated in Figure 15. Alternatively, the purified material 220 is not transferred to the original container, but to another container adapted to perform steps 805-815.

Although not represented in Figure 15 for simplification purposes, after step 820, the original container can be refilled at any time with either pure material 220 (which has not been used yet and does not contain sulfur) or purified material 220 (which has been purified according to steps 825-835 and contains substantially no sulfur, e.g. only at a concentration below a predetermined threshold). After the refilling, steps 805-815 are repeated. This allows to operate the original container in sulfur capture (desulfurization) mode almost continuously, except for the operations of transferring the material saturated with sulfur according to step 820 and refilling the original container with pure or purified material.

Although the present disclosure has been described hereinabove by way of non-restrictive, illustrative embodiments thereof, these embodiments may be modified at will within the scope of the appended claims without departing from the spirit and nature of the present disclosure.

## Claims

1. A system adapted for performing a regenerative desulfurization of raw biogas, the system comprising:
a container (210) containing a material (220) adapted for capturing and converting into solid sulfur hydrogen sulfide;
means for injecting the raw biogas comprising the hydrogen sulfide in the container (210);
means for extracting biogas without hydrogen sulfide from the container (210), the hydrogen sulfide being captured and converted into the solid sulfur by the material (220);
means for injecting a regeneration gas heated at a predetermined temperature in the container (210), the predetermined temperature allowing extraction of the sulfur from the material (220) in the form of one or more sulfur compounds; and
means for extracting the regeneration gas and the one or more sulfur compounds from the container (210).

2. The system of claim 1, further comprising a control device (600) adapted for collecting (710) data representative of operating conditions of the system, executing (720) a control algorithm (612) using the collected data for determining at least one operating parameter of a component of the system, and applying (730) the at least one operating parameter to the component.

3. The system of claim 2, the operating parameter comprises at least one of the following: initiation or interruption of the injection of the raw biogas with hydrogen sulfide in the container (210), flow rate of the raw biogas with hydrogen sulfide injected in the container (210), initiation or interruption of the extraction of biogas without hydrogen sulfide from the container (210), flow rate of the biogas without hydrogen sulfide extracted from the container (210), initiation or interruption of the injection of heated regeneration gas in the container (210), flow rate of the heated regeneration gas injected in the container (210), initiating or stopping the extraction of the regeneration gas and the one or more sulfur compounds from the container (210).

4. The system of claim 2, wherein the data representative of operating conditions comprise at least one of the following: flow rate of raw biogas with hydrogen sulfide injected in the container (210), concentration in at least one of hydrogen sulfide, oxygen, ammonia, methane, carbon dioxide and humidity of the raw biogas injected in the container (210), flow rate of biogas extracted from the container (210), concentration in hydrogen sulfide of the biogas extracted from the container (210), estimation of a concentration of sulfur in the material (220), flow rate of regeneration gas injected in the container (210), concentration in nitrogen of the regeneration gas injected in the container (210), concentration in oxygen of the regeneration gas injected in the container (210), temperature of the regeneration gas injected in the container (210), flow rate of the one or more sulfur compound extracted from the container (210), concentration of the one or more sulfur compound extracted from the container (210), quantity of solid sulfur extracted from the container (210), concentration in oxygen of the regeneration gas extracted from the container (210), concentration in other exhaust gas extracted from the container (210), temperature inside the container (210), pressure inside the container (210), humidity inside the container (210), and weather conditions.

5. The system of claim 1 comprising a first container (210) consisting of the aforementioned container (210), the system further comprising:
a second container (210') containing the material (220) adapted for capturing and converting into solid sulfur the hydrogen sulfide;
means for injecting the raw biogas comprising the hydrogen sulfide in the second container (210');
means for extracting biogas without hydrogen sulfide from the second container (210'), the hydrogen sulfide being captured and converted into the solid sulfur by the material (220) in the second container (210');
means for injecting the regeneration gas heated at the predetermined temperature in the second container (210'), the predetermined temperature allowing extraction of the sulfur from the material (220) in the second container (210') in the form of one or more sulfur compounds;
means for extracting the regeneration gas and the one or more sulfur compounds from the second container (210');
wherein the raw biogas with hydrogen sulfide is injected in the container (210) while the regeneration gas heated at the predetermined temperature is injected in the second container (210'), and the raw biogas with hydrogen sulfide is injected in the second container (210') while the regeneration gas heated at the predetermined temperature is injected in the first container (210).

6. The system of claim 1, wherein the material (220) is a porous carbonaceous solid material impregnated with metallic nanoparticles and the regeneration gas comprises nitrogen mixed with at least one of oxygen and carbon dioxide.

7. A method for performing a regenerative desulfurization procedure of raw biogas, the method comprising:
(i) injecting (505) the raw biogas comprising hydrogen sulfide in a container (210), the container (210) containing a material (220) adapted for capturing and converting into solid sulfur the hydrogen sulfide;
(ii) extracting (510) biogas without hydrogen sulfide from the container (210), the hydrogen sulfide being captured and converted into the solid sulfur by the material (220);
(iii) stopping (515) the injection of the raw biogas comprising the hydrogen sulfide in the container (210);
(iv) injecting (520) a regeneration gas heated at a predetermined temperature in the container (210), the predetermined temperature allowing extraction of the sulfur from the material (220) in the form of one or more sulfur compounds;
(v) extracting (525) the regeneration gas and the one or more sulfur compounds from the container (210); and
(vi) stopping (530) the injection of the regeneration gas in the container (210).

8. The method of claim 7, wherein steps (i) to (vi) are repeated several times.

9. The method of claim 7, further comprising collecting (710) by a control device (600) data representative of operating conditions of the regenerative desulfurization procedure, executing (720) by the control device (600) a control algorithm (612) using the collected data for determining at least one operating parameter of the regenerative desulfurization procedure, and applying (730) the at least one operating parameter to a component implementing the regenerative desulfurization procedure.

10. The method of claim 9, wherein the operating parameter comprises at least one of the following: initiation or interruption of the injection of the raw biogas with hydrogen sulfide in the container (210), flow rate of the raw biogas with hydrogen sulfide injected in the container (210), initiation or interruption of the extraction of biogas without hydrogen sulfide from the container (210), flow rate of the biogas without hydrogen sulfide extracted from the container (210), initiation or interruption of the injection of heated regeneration gas in the container (210), flow rate of the heated regeneration gas injected in the container (210), initiating or stopping the extraction of the regeneration gas and the one or more sulfur compounds from the container (210).

11. The method of claim 9, wherein the data representative of operating conditions comprise at least one of the following: flow rate of raw biogas with hydrogen sulfide injected in the container (210), concentration in at least one of hydrogen sulfide, oxygen, ammonia, methane, carbon dioxide and humidity of the raw biogas injected in the container (210), flow rate of biogas extracted from the container (210), concentration in hydrogen sulfide of the biogas extracted from the container (210), estimation of a concentration of sulfur in the material (220), flow rate of regeneration gas injected in the container (210), concentration in nitrogen of the regeneration gas injected in the container (210), concentration in oxygen of the regeneration gas injected in the container (210), temperature of the regeneration gas injected in the container (210), flow rate of the one or more sulfur compound extracted from the container (210), concentration of the one or more sulfur compound extracted from the container (210), quantity of solid sulfur extracted from the container (210), concentration in oxygen of the regeneration gas extracted from the container (210), concentration in other exhaust gas extracted from the container (210), temperature inside the container (210), pressure inside the container (210), humidity inside the container (210), and weather conditions.

12. The method of claim 7, further comprising collecting (710) by a control device (600) data representative of operating conditions of the regenerative desulfurization procedure, determining (720) by the control device (600) during the execution of steps (i) and (ii) based on the collected data that the material (220) is saturated with sulfur, and enforcing (730) by the control device (600) the interruption of steps (i) and (ii) and the initiation of steps (iii), (iv) and (v).

13. The method of claim 7, further comprising collecting (710) by a control device (600) data representative of operating conditions of the regenerative desulfurization procedure, determining (720) by the control device (600) during the execution of steps (iv) and (v) based on the collected data that regeneration of the material (220) is completed, and enforcing (730) by the control device (600) the interruption of steps (iv) and (v) and the initiation of steps (vi), (i) and (ii).

14. The method of claim 7, further comprising collecting (710) by a control device (600) data representative of operating conditions of the regenerative desulfurization procedure, and determining (720) by the control device (600) based on the collected data that material (220) needs to be added to the container (210) to replace material (220) consumed by the regenerative desulfurization procedure.

15. The method of claim 7, further comprising:
simultaneously to steps (i) and (ii),
injecting (520) the regeneration gas heated at the predetermined temperature in a second container (210'), the second container (210') containing the material (220) with deposited sulfur; and
extracting (525) from the second container (210') the regeneration gas and one or more sulfur compounds generated by the extraction of the sulfur from the material (220) of the second container (210') under the action of the heated regeneration gas; and
simultaneously to steps (iv) and (v),
injecting (505) the raw biogas comprising the hydrogen sulfide in the second container (210'), the second container (210') containing the material (220) adapted for capturing and converting into solid sulfur the hydrogen sulfide; and
extracting (510) biogas without hydrogen sulfide from the second container (210'), the hydrogen sulfide being captured and converted into the solid sulfur by the material (220) of the second container (210').
